# EUROPÄISCHE PATENTSCHRIFT

(11) **EP 4 178 471 B1**
(45) Veröffentlichungstag und Bekanntmachung des Hinweises auf die Patenterteilung: **31.12.2025**
(21) Anmeldenummer: 21746655.6
(22) Anmeldetag: 05.07.2021
(51) Int. Cl.: A61B 17/29

(54) **MEDIZINISCHES INSTRUMENT UND VERFAHREN**
MEDICAL INSTRUMENT AND METHOD
INSTRUMENT MÉDICAL ET PROCÉDÉ

(30) Priorität: 08.07.2020 DE 102020117962
(43) Veröffentlichungstag der Anmeldung: 17.05.2023
(73) Patentinhaber: Karl Storz SE & Co. KG, 78532 Tuttlingen (DE)
(72) Erfinder: BLOCHER, Martin, 78532 Tuttlingen (DE); GRÜNER, Sven Axel, 78532 Tuttlingen (DE); HOLZER, Judith, 78532 Tuttlingen (DE); KÄRCHER, Daniel, 78532 Tuttlingen (DE); LÄNGLE, Dominik, 78532 Tuttlingen (DE); MERZ, Robin, 78532 Tuttlingen (DE); SCHNEIDER, Janosz, 78532 Tuttlingen (DE); SCHNEIDER, Sven, 78532 Tuttlingen (DE); STEFAN, Jochen, 78532 Tuttlingen (DE); UNGER, Tobias, 78532 Tuttlingen (DE); WAGNER, Sebastian, 78532 Tuttlingen (DE)
(86) Internationale Anmeldenummer: PCT/EP2021/068525
(87) Internationale Veröffentlichungsnummer: WO 2022/008453

(56) Entgegenhaltungen:
- EP-A1- 2 564 794
- DE-U1- 9 213 119
- US-A- 5 174 300
- US-A1- 2004 167 569
- US-A1- 2009 177 039

## Beschreibung

Die Erfindung betrifft ein medizinisches Instrument, mit einem langerstreckten Schaft, einem beweglichen Werkzeug an einem distalen Ende des Schaftes, einer Handhabe mit einem beweglichen Griffteil an einem proximalen Ende des Schaftes, und mit einem in seiner Längsrichtung beweglichen Kraftübertragungselement, das mit dem beweglichen Griffteil und mit dem Werkzeug in Wirkverbindung steht, so dass eine Betätigung des beweglichen Griffteils in eine Bewegung des Werkzeugs umgesetzt wird.

Die Erfindung betrifft ferner ein Verfahren zum Immobilisieren und Freigeben der Beweglichkeit eines beweglichen Werkzeuges eines medizinischen Instruments.

Aus der Firmenbroschüre STORZ - THE WORLD OF ENDOSCOPY, Band LAPAROSCOPY IN SURGERY, GYNECOLOGY, UROLOGY 8. Ausgabe 2/2016/US, sind medizinische Instrumente bekannt, die bspw. als chirurgische Schneid- oder Fasszangen ausgebildet sind. Eine chirurgische Zange weist einen langerstreckten Schaft, ein Werkzeug mit einem oder mehreren Maulteilen zum Schneiden oder Fassen am distalen Ende des Schaftes und eine Handhabe mit einem beweglichen Griffteil am proximalen Ende des Schaftes auf. Mittels eines Kraftübertragungselementes, das sich in Längsrichtung des Schaftes erstreckt, wird eine Betätigung des beweglichen Griffteils in eine Bewegung des einen oder der mehreren Maulteile übertragen, bspw. um das Werkzeug des Instruments zu öffnen oder zu schließen.

Beispielsweise bei Fasszangen, die Maulteile aufweisen, von denen zumindest eines beweglich ist, ist es häufig wünschenswert, dass der Operateur, wenn er mittels Betätigung des beweglichen Griffteils die Maulteile so weit geschlossen hat, dass das zu fassende Objekt sicher gefasst ist, das bewegliche Griffteil loslassen oder zumindest die Handkraft reduzieren kann, ohne dass sich die Maulteile wieder so weit öffnen, dass das gefasste Objekt von dem Werkzeug abfällt.

Zu diesem Zweck ist bei den bekannten Instrumenten an der Handhabe zwischen den Griffteilen ein Rastgesperre angeordnet, das eine Anzahl von Rastzähnen aufweist, die eine Immobilisierung des beweglichen Griffteils in Öffnungsrichtung des Werkzeuges ermöglichen. Durch die Immobilisierung des beweglichen Griffteiles ist die Längsbewegung des Kraftübertragungselementes in der Bewegungsrichtung, die dem Öffnen des Werkzeugs entspricht, gesperrt, und das Werkzeug kann sich nicht unerwünscht öffnen.

Ein Rastgesperre zum Immobilisieren des Werkzeugs des Instruments hat jedoch den Nachteil, dass eine Rastung nur in vordefinierten Abständen, die durch den Abstand benachbarter Zähne vorgegeben sind, möglich ist. Dies kann bspw. dazu führen, dass der Operateur das bewegliche Griffteil weiter als zum sicheren Fassen des Objektes erforderlich bewegen muss, um den nächsten Rastpunkt zu erreichen. Dies kann wiederum dazu führen, dass das zu fassende Objekt, bspw. eine chirurgische Nadel, mit übererhöhter Kraft von den Maulteilen gefasst ist und dadurch geschädigt werden kann. Außerdem kann im Kraftübertragungselement eine übermäßige Spannung auftreten, die zu einer Beschädigung des Kraftübertragungselements führen kann. Eine Verrastung des beweglichen Griffteils unter hoher Spannung des Kraftübertragungselements kann zu einem Reißen des Kraftübertragungselements führen.

Aus EP 2 564 794 A1 ist ein medizinisches Instrument bekannt, dessen Schaft um seine Längsachse relativ zur Handhabe drehbar ist. Bei diesem Instrument ist eine Federanordnung vorhanden, die eine Schenkelfeder aufweist, die um ein grifffestes Element geschlungen ist, um den Schaft und die Handhabe relativ zueinander drehfest zu halten, wobei eine Drehbewegung jedoch möglich ist, wenn ein Betätigungselement zum Drehen des Schaftes betätigt wird.

Auch die Patentanmeldung US 2009/0177039 A1 offenbart ein medizinisches Instrument mit einem um seine Längsachse rotierbaren Schaft. Zum Rotieren des Schafts ist an der Handhabe ein Betätigungselement angebracht. Ein Reibelement am proximalen Ende des Schafts verhindert dessen Rotation, es sei denn, dass ein Drehmoment vom Betätigungselement aus aufgebracht wird. US 2009/0177039 A1 zeigt ebenfalls ein chirurgisches Instrument mit rotierbaren Maulteilen. Die Maulteile lassen sich vom Handgriff aus rotieren, nicht aber ausgehend vom distalen Ende des Instruments

Das Patent US 5,588,581 A lehrt die Verwendung einer Schenkelfeder in einem Handgriff, um dessen zusammendrückbare Griffteile in ihre gespreizte Ausgangsposition zurückzuführen.

Die Patentanmeldung US 2004/0167569 A1 offenbart ein medizinisches Instrument mit einem Verriegelungs- und Freigabemechanismus, wobei dieser die Bewegung eines Kraftübertragungselements in der einen Richtung verriegelt und in einer entgegengesetzten Richtung freigibt

Die Patentschrift US 5,174,300 A lehrt ein chirurgisches Instrument rotierbaren Endeffektoren und einem Verriegelungselement zum Verriegeln und Freigeben der Endeffektor-Rotation.

Das Gebrauchsmuster DE 92 13 119 U1 offenbart ein chirurgisches Instrument mit einer Umlaufsperre. Der Handgriff umfasst zwei Griffbranchen, von denen eine zweigeteilt ist. Der Erfindung liegt die Aufgabe zugrunde, ein medizinisches Instrument der eingangs genannten Art mit einer stufenlosen Immobilisierung der Beweglichkeit des Werkzeugs bereitzustellen.

Des Weiteren liegt der Erfindung die Aufgabe zugrunde, ein Verfahren zum Immobilisieren und Freigeben der Beweglichkeit eines beweglichen Werkzeuges eines medizinischen Instruments anzugeben.

Erfindungsgemäß wird die zuerst genannte Aufgabe durch ein medizinisches Instrument gelöst, mit einem langerstreckten Schaft, einem beweglichen Werkzeug an einem distalen Ende des Schaftes, einer Handhabe mit einem beweglichen Griffteil an einem proximalen

Ende des Schaftes, und mit einem in seiner Längsrichtung beweglichen Kraftübertragungselement, das mit dem beweglichen Griffteil und mit dem Werkzeug in Wirkverbindung steht, so dass eine Betätigung des beweglichen Griffteils in eine Bewegung des Werkzeugs umgesetzt wird, und mit einer an der Handhabe angeordneten Federanordnung, die zumindest eine Schenkelfeder, die zumindest eine Windung und zwei Schenkel aufweist, und einen Zapfen aufweist, wobei sich die zumindest eine Windung um den Zapfen herum erstreckt, wobei das bewegliche Griffteil bei seiner Betätigung auf zumindest einen der Schenkel der Schenkelfeder eine Kraft ausübt, die die Reibung zwischen der Windung und dem Zapfen verringert, so dass die Schenkelfeder um den Zapfen drehbeweglich ist und sich das Kraftübertragungselement und das Werkzeug bewegen können, wogegen die Schenkelfeder bei einer vom Werkzeug auf das Kraftübertragungselement in dessen Längsrichtung übertragenen Kraft um den Zapfen nicht drehbeweglich ist, so dass sich das Kraftübertragungselement und das Werkzeug nicht bewegen können.

Bei dem erfindungsgemäßen medizinischen Instrument ist eine stufenlose Selbsthemmung für das Werkzeug realisiert, die jeglicher vom Werkzeug ausgehender Krafteinwirkung auf das Kraftübertragungselement instantan entgegenwirkt und eine Bewegung des Werkzeugs unterbindet, wenn eine Bewegung des Werkzeugs nicht durch Betätigung des beweglichen Griffteils gewollt aktuiert ist. Ist das Werkzeug bspw. als Fasswerkzeug ausgestaltet, und wird mit dem Werkzeug ein Objekt gefasst, übt das Objekt auf das Werkzeug eine Kraft in Öffnungsrichtung des Werkzeugs aus. Diese von dem Objekt ausgeübte Kraft überträgt sich auf das Kraftübertragungselement, das sich ohne die erfindungsgemäß vorgesehene Selbsthemmung in Längsrichtung bewegen würde, wenn der Operateur das bewegliche Griffteil loslässt. Bei dem erfindungsgemäßen Instrument setzt jedoch beim Loslassen des Griffteils unmittelbar eine Selbsthemmung der Bewegung des Werkzeugs aufgrund des Reibschlusses zwischen der zumindest einen Windung der Schenkelfeder und dem Zapfen ein. Die vom Werkzeug auf das Kraftübertragungselement übertragene Kraft kann die Reibung zwischen der zumindest einen Windung der Schenkelfeder und dem Zapfen sogar erhöhen, indem sich die Windung noch enger um den Zapfen schlingt. Die Selbsthemmung tritt in jeder Stellung des Werkzeugs automatisch ein, wenn das bewegliche Griffteil nicht betätigt wird, um das Werkzeug beabsichtigt zu bewegen. Die Selbsthemmung des erfindungsgemäßen Instruments ist vorzugsweise über den gesamten möglichen Bewegungsweg des Werkzeugs stufenlos. Wenn das bewegliche Griffteil betätigt wird, wird dagegen die Reibung zwischen der zumindest einen Windung der Schenkelfeder und dem Zapfen verringert, so dass sich die Schenkelfeder um den Zapfen drehen kann. Eine Verringerung der Reibung kann auch eine vollständige Aufhebung der Reibung umfassen. Der Selbsthemmungsmechanismus des erfindungsgemäßen Instruments ist somit nicht nur stufenlos, sondern weist noch den weiteren Vorteil auf, dass kein eigens zu bedienender Lösemechanismus zum Lösen der Selbsthemmung erforderlich ist, was die Bedienung des medizinischen Instruments vereinfacht und auch den strukturellen Aufwand des Selbsthemmungsmechanismus gering hält.

Die erfindungsgemäße Ausgestaltung des medizinischen Instruments ist nicht nur für Fasszangen oder Fassinstrumente vorteilhaft, sondern auch für Schneidzangen oder Schneidinstrumente. Bei einem Schneidinstrument ist das Werkzeug als Schneidwerkzeug ausgebildet, und weist beispielsweise zwei miteinander schneidend wirkende Maulteile auf. Der erfindungsgemäße Selbsthemmungsmechanismus kann beispielsweise ein nicht gewolltes Schließen des Werkzeugs verhindern, d.h. ein Schließen des Werkzeugs kann nur durch Betätigen des beweglichen Griffteils bewirkt werden.

Die zumindest eine Windung der Schenkelfeder kann vorzugsweise bereits im Ruhezustand so eng um den Zapfen geschlungen sein, dass die Schenkelfeder im Ruhezustand aufgrund Reibung um den Zapfen nicht drehbeweglich ist.

Hierbei ist von Vorteil, dass die Selbsthemmung bereits bei kleinsten vom Werkzeug auf das Kraftübertragungselement übertragenen Kräften wirksam wird, wodurch eine Selbsthemmung stets gewährleistet ist.

Der Selbsthemmungsmechanismus kann auch so ausgebildet sein, dass eine vom Werkzeug auf das Kraftübertragungselement in dessen Längsrichtung übertragene Kraft an zumindest einem der Schenkel angreift, wodurch die Reibung zwischen der zumindest einen Windung und dem Zapfen erhöht wird.

In dieser Ausgestaltung wirkt eine vom Werkzeug auf das Kraftübertragungselement übertragene Kraft auf den oder die Schenkel der Schenkelfeder reibungserhöhend, indem sich die Windung noch enger um den Zapfen schlingt.

Grundsätzlich kann es vorgesehen sein, dass die Selbsthemmung entweder nur in Schließrichtung des Werkzeugs oder nur in Öffnungsrichtung des Werkzeugs wirkt. Vorzugsweise kann die Selbsthemmung jedoch sowohl in Öffnungs- als auch in Schließrichtung des Werkzeugs wirksam sein.

So kann vorgesehen sein, dass die Längsbewegung des Kraftübertragungselements und die Bewegung des Werkzeugs sowohl gesperrt ist, wenn die über das Werkzeug auf das Kraftübertragungselement wirkende Kraft nach distal gerichtet ist, als auch, wenn die über das Werkzeug auf das Kraftübertragungselement wirkende Kraft nach proximal gerichtet ist.

Mit anderen Worten wird die Selbsthemmung stets wirksam, unabhängig davon, ob die vom Werkzeug übertragene Kraft das Kraftübertragungselement nach distal zu ziehen oder nach proximal zu schieben versucht.

Ebenso kann die freie Gängigkeit der Längsbewegung des Kraftübertragungselements in Richtung nach distal als auch nach proximal frei sein, um das Werkzeug zu bewegen, wenn das bewegliche Griffteil in seinen beiden Betätigungsrichtungen bewegt wird. Hierzu ist vorzugsweise vorgesehen, dass in den beiden Betätigungsrichtungen des beweglichen Griffteils an jeweils einem der Schenkel der Schenkelfeder eine Kraft angreift, die die Reibung zwischen der Windung und dem Zapfen verringert.

Die Selbsthemmung und die freie Gängigkeit der Werkzeugbewegung können somit jeweils bidirektional wirksam sein.

Wenn das bewegliche Griffteil um eine Drehachse verschwenkbar ist, wie in einer Ausgestaltung vorgesehen ist, fluchtet der Zapfen vorzugsweise mit der Drehachse.

Hierdurch wird zum einen eine platzsparende Anordnung der Federanordnung realisiert. Zum anderen ergeben sich günstige Hebelverhältnisse, um durch Betätigen des beweglichen Griffteils auf den oder die Schenkel eine Kraft auszuüben, die die Reibung zwischen der zumindest einen Windung der Schenkelfeder und dem Zapfen verringert.

In einer konstruktiv einfachen Ausgestaltung weist das bewegliche Griffteil einen Mitnehmer aufweist, der bei Betätigung des beweglichen Griffteils auf zumindest einen der Schenkel der Schenkelfeder die Kraft ausübt, um die Reibung zwischen der Windung und dem Zapfen zu verringern. Der Mitnehmer kann beispielsweise den einen Schenkel relativ zu dem anderen Schenkel bewegen, so dass sich der Durchmesser der Windung vergrößert und dadurch die Reibung mit dem Zapfen verringert wird.

Vorzugsweise stehen die Schenkel der Schenkelfeder von der zumindest einen Windung zur gleichen Seite ab, und der Mitnehmer ist zwischen den Schenkeln der Schenkelfeder angeordnet.

Bei einer Bewegung des beweglichen Griffteils in einer Betätigungsrichtung kann der Mitnehmer an einem ersten der beiden Schenkel der Schenkelfeder angreifen und dieses von dem zweiten Ende der Schenkelfeder wegbewegen, wodurch die Reibung zwischen der Federwindung und dem Zapfen beispielsweise verringert wird. Wird das bewegliche Griffteil in der entgegengesetzten Bewegungsrichtung betätigt, kann der Mitnehmer das zweite Ende der Schenkelfeder vom ersten Ende der Schenkelfeder wegbewegen, wodurch wiederum die Reibung zwischen der Federwindung und dem Zapfen beispielsweise verringert wird. Die Schenkel können in Längsrichtung des Schaftes gesehen axial hintereinander angeordnet sein, während sich der Mitnehmer in einer Ebene, die die Längsachse des Schaftes enthält, quer zur Längsrichtung des Schaftes erstreckt.

Das bewegliche Griffteil kann eine Aufnahme zur Verbindung eines proximalen Endes des Kraftübertragungselements mit dem beweglichen Griffteil aufweisen, und zumindest ein Schenkel, vorzugsweise beide Schenkel, können mit der Aufnahme direkt oder indirekt fest verbunden sein.

Wenn vom Werkzeug auf das Kraftübertragungselement eine Kraft wirkt, die das Kraftübertragungselement in distale oder proximale Richtung zu bewegen versucht, überträgt sich diese Kraft von der Aufnahme auf die Schenkel der Schenkelfeder, ohne dass die Reibung der Federwindung mit dem Zapfen verringert wird und somit die Selbsthemmung wirksam ist. Eine solche Kraft kann aber auch die Windung der Schenkelfeder enger um den Zapfen schlingen und somit die Reibung zwischen der Windung und dem Zapfen erhöhen.

Die Federanordnung kann zumindest zwei Schenkelfedern aufweisen, die vorzugsweise beidseits einer Längsmittelachse des Schaftes angeordnet sind.

Aus Platzgründen im Bereich des Griffs ist die außermittige Anordnung der Federanordnung vorteilhaft. Die beidseitige Anordnung der Federanordnung in Bezug auf die Längsmittelachse hat den Vorteil einer verbesserten Wirkung der Selbsthemmung aufgrund einer durch zumindest zwei Federwindungen erhöhten Reibung sowie einer symmetrischen Lastverteilung des Selbsthemmungsmechanismus. Der Zapfen kann sich beidseits der Längsmittelachse des Schaftes erstrecken, oder es ist jeweils ein Zapfen auf beiden Seiten der Längsmittelachse vorhanden.

Die Federanordnung kann auch mehr als zwei Schenkelfedern, beispielsweise vier Schenkelfedern aufweisen, die paarweise beidseits der Längsmittelachse des Schaftes angeordnet sind.

Die selbsthemmende Wirkung der Federanordnung verbessert sich mit der Anzahl an Schenkelfedern, weil durch eine höhere Anzahl von Schenkelfedern die Reibung zwischen den Windungen der Schenkelfedern und den Zapfen bzw. den Zapfen größer wird.

Alternativ oder zusätzlich kann die zumindest eine Schenkelfeder mehrere Windungen aufweisen. Auch hierdurch kann eine höhere Reibung zwischen der Schenkelfeder und dem Zapfen hergestellt werden.

Des Weiteren kann die Federanordnung eine Verwindungssicherung aufweisen, die eine Stabilisierung der Schenkelfeder(n) gegen Verwindung gewährleistet.

Weiterhin wird ein Verfahren zum Immobilisieren und Freigeben der Beweglichkeit eines beweglichen Werkzeuges eines medizinischen Instruments bereitgestellt, das einen langerstreckten Schaft, das bewegliche Werkzeug an einem distalen Ende des Schaftes, eine Handhabe mit einem beweglichen Griffteil an einem proximalen Ende des Schaftes und ein in seiner Längsrichtung bewegliches Kraftübertragungselement aufweist, das mit dem beweglichen Griffteil und mit dem Werkzeug in Wirkverbindung steht, wobei die Beweglichkeit des Werkzeuges und des beweglichen Griffteils unmittelbar unterbunden wird, wenn das bewegliche Griffteil nicht betätigt wird, und wobei die Beweglichkeit des Werkzeuges unmittelbar durch Betätigen des beweglichen Griffteils freigegeben wird.

Das Verfahren hat die gleichen Vorteile wie das erfindungsgemäße Instrument.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine Seitenansicht eines medizinischen Instruments in einer Prinzipdarstellung;
- Fig. 2: einen Ausschnitt einer Handhabe mit einem Selbsthemmungsmechanismus, wie er bei einem Instrument in Fig. 1 implementiert sein kann, in perspektivischer Darstellung;
- Fig. 3: eine Seitenansicht der Anordnung in Fig. 2;
- Fig. 4: einen vergrößerten Ausschnitt der Anordnung in Fig. 3;
- Fig. 5: eine Ansicht von proximal auf die Anordnung in Fig. 2; und
- Fig. 6: eine Explosionszeichnung der Anordnung in Fig. 2 in perspektivischer Darstellung.

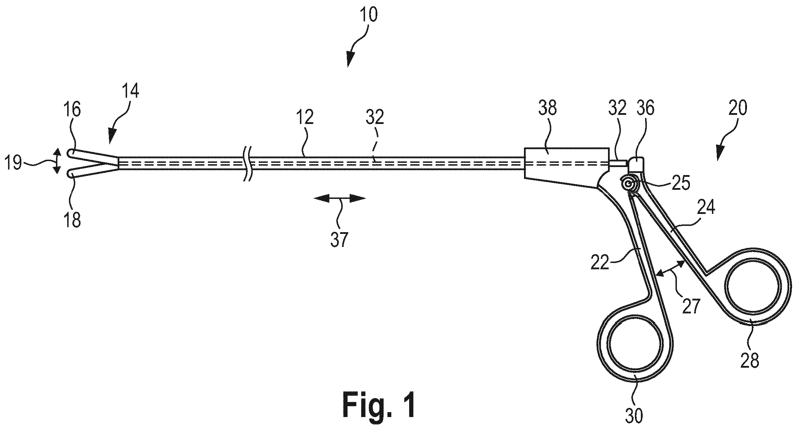

Fig. 1 zeigt ein mit dem allgemeinen Bezugszeichen 10 versehenes medizinisches Instrument. Das medizinische Instrument 10 kann insbesondere eine chirurgische Zange sein. Die chirurgische Zange kann zum Schneiden, bspw. von Gewebe, oder zum Fassen von Gewebe oder anderen Objekten, wie bspw. einer chirurgischen Nadel, eines Implantats über dergleichen, ausgebildet sein.

Allgemein weist das Instrument 10 einen langerstreckten Schaft 12 auf, an dessen distalem Ende ein Werkzeug 14 angeordnet ist. Das Werkzeug 14 kann ein erstes Maulteil 16 und ein zweites Maulteil 18 aufweisen. Zumindest eines der beiden Maulteile 16, 18 ist beweglich, bspw. verschwenkbar, am distalen Ende des Schaftes angeordnet. Die Maulteile 16 und 18 können, wie mit einem Doppelpfeil 19 angedeutet ist, somit aufeinander zu bewegt werden, um das Werkzeug 14 schließen, und sie können voneinander wegbewegt werden, um das Werkzeug 14 zu öffnen.

Das Werkzeug 14 kann in anderen Ausgestaltungen bspw. als Werkzeug zum Zurückhalten von Organen oder Gewebe oder als Spatel ausgebildet sein. Die Ausgestaltung des Werkzeuges 14 mit Maulteilen 16 und 18 ist daher nur beispielhaft. Eine Bewegung des Werkzeuges 14 kann auch in einer translatorischen Bewegung des Werkzeuges 14 bestehen.

Am proximalen Ende des Schaftes 12 ist eine Handhabe 20 angeordnet. Die Handhabe 20 weist ein festes Griffteil 22 und ein bewegliches Griffteil 24 auf. In anderen Ausgestaltungen, die hier nicht gezeigt sind, können jedoch auch beide Griffteile 22 und 24 beweglich sein. Das bewegliche Griffteil 24 ist um eine Drehachse 25 verschwenkbar beweglich gelagert, wie mit einem Doppelpfeil 27 angedeutet ist.

Das Griffteil 22 und das Griffteil 24 sind in dem Ausführungsbeispiel mit jeweils einem Fingerring 28, 30 ausgestattet, so dass die Handhabe 20 mit zwei Fingern einer selben Hand gehalten und betätigt werden kann. Die hier gezeigte Ausgestaltung der Handhabe 20 als Scherengriffanordnung ist jedoch nur beispielhaft. In anderen Ausgestaltungen kann die Handhabe 20 bspw. als Stabgriffanordnung ausgebildet sein, die von einer Hand umgriffen und betätigt werden kann.

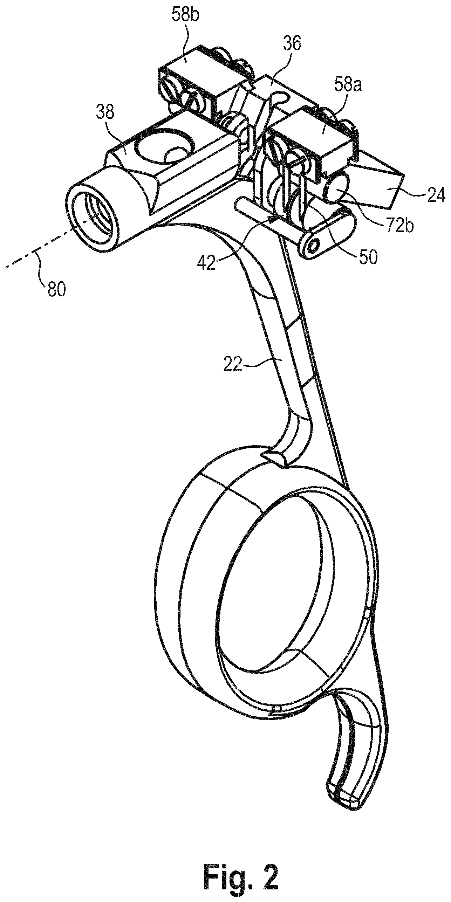

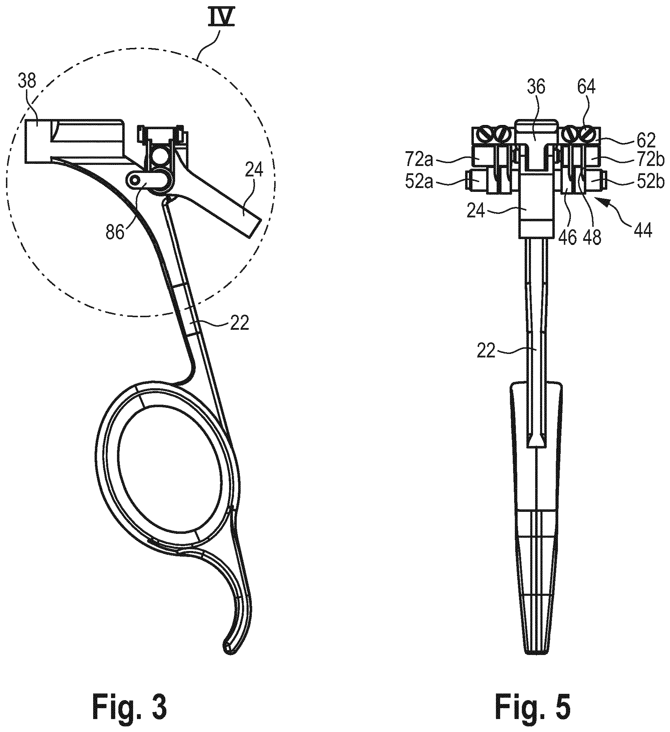

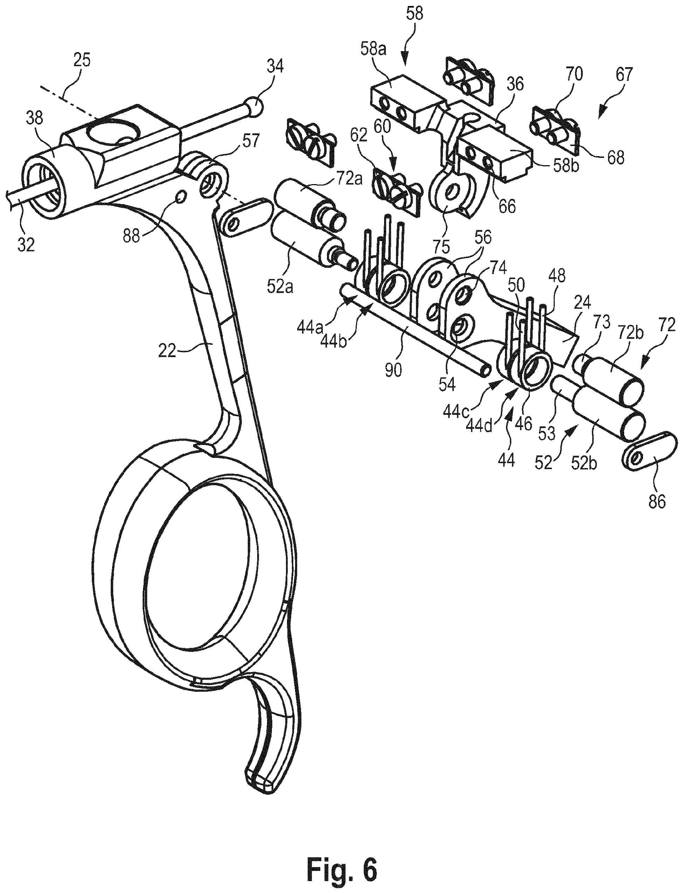

Das Instrument 10 weist weiterhin ein langerstrecktes Kraftübertragungselement 32 auf, das sich in Längsrichtung des Schaftes 12, üblicherweise im Inneren des Schaftes 12, von der Handhabe 20 bis zu dem Werkzeug 14 erstreckt. Das Kraftübertragungselement 32 weist, wie in Fig. 5 gezeigt ist, einen bspw. kugelförmig ausgebildeten Kopf 34 auf, der in einer entsprechenden Aufnahme 36 (siehe auch Fig. 2 und 6) am beweglichen Griffteil 24 aufgenommen ist. Die Aufnahme 36 ist entsprechend der Form des Kopfes 34 komplementär zu dem Kopf 34 ausgebildet, im vorliegenden Ausführungsbeispiel kann die Aufnahme 36 entsprechend als Kugelpfanne ausgebildet sein.

Am distalen Ende steht das Kraftübertragungselement 32, das auch als Zug- und Druckstange bezeichnet werden kann, mit dem Werkzeug 14 in Wirkverbindung. Das Kraftübertragungselement 32 ist in Richtung seiner Längserstreckung längsbeweglich, wobei die Längsbeweglichkeit dem Bewegen des Werkzeugs 14 dient. Die Längsbeweglichkeit des Kraftübertragungselements 32 ist in Fig. 1 mit einem Doppelpfeil 37 angedeutet. Wenn bspw. das bewegliche Griffteil 24 zu dem feststehenden Griffteil 22 hin verschwenkt wird, wird das Kraftübertragungselement 32 nach proximal bewegt. Diese nach proximal gerichtete Längsbewegung des Kraftübertragungselements 32 dient dem Bewegen des Werkzeuges in eine erste Richtung, im vorliegenden Ausführungsbeispiel dem Öffnen des Werkzeugs 14. Die umgekehrte Verschwenkbewegung des beweglichen Griffteils 24 schiebt das Kraftübertragungselement 32 in Längsrichtung nach distal, wobei diese Längsbewegung des Kraftübertragungselements 32 der entgegengesetzten Bewegung des Werkzeuges 14 dient, hier dem Schließen des Werkzeuges 14. Diese Art der Aktuatorik ist jedoch nur beispielhaft. Beispielsweise kann in anderen Ausgestaltungen die nach proximal gerichtete Bewegung des Kraftübertragungselements 32 dem Öffnen des Werkzeuges 14 dienen, und die nach distal gerichtete Längsbewegung des Kraftübertragungselements 32 dem Schließen des Werkzeugs 14.

Die Handhabe 20 weist ferner ein Gehäuse 38 zur Befestigung des Schaftes an der Handhabe 20 auf.

Mit Bezug auf Fig. 2 bis 6 wird nachfolgend ein Selbsthemmungsmechanismus 40 beschrieben, der verhindert, dass sich das Werkzeug 14 bei einer auf das Werkzeug 14 wirkenden Kraft bewegt, während bei einer Betätigung des beweglichen Griffteils 24 die Bewegung des Werkzeuges 14 frei ist. Der Selbsthemmungsmechanismus 40 kann bei dem Instrument 10 in Fig. 1 implementiert sein. In Fig. 2 bis 6 werden daher die gleichen Bezugszeichen wie in Fig. 1 für Elemente oder Teile verwendet, die Teilen oder Elementen des Instruments 10 in Fig. 1 entsprechen. Fig. 2 bis 6 zeigen nur die Handhabe 20, wobei das bewegliche Griffteil 24 nur ausschnittsweise gezeigt ist. Der Schaft 12 und das Werkzeug 14 sind aus Gründen der Übersichtlichkeit weggelassen. Das Kraftübertragungselement 32 ist in Fig. 5 in seinem proximalen Bereich gezeigt.

Der an der Handhabe 20 angeordnete Selbsthemmungsmechanismus 40 weist eine Federanordnung 42 auf. Die Federanordnung 42 weist zumindest eine Schenkelfeder 44 auf. In dem vorliegenden Ausführungsbeispiel weist die Federanordnung 42 insgesamt vier Schenkelfedern 44a, 44b, 44c und 44d auf. Die Schenkelfedern 44a, 44b, 44c und 44d können untereinander gleich sein. Nachfolgend werden die einzelnen Schenkelfedern 44a, 44b, 44c und 44d einheitlich auch als Schenkelfeder 44 bezeichnet.

Die Schenkelfeder 44, die auch als Torsionsfeder bezeichnet werden kann, ist beispielsweise aus Federdraht gebildet. Die Schenkelfeder 44 weist zumindest eine Windung 46 und zwei Schenkel 48, 50 auf. Unter zumindest einer Windung ist zu verstehen, dass sich die Schenkelfeder im gewundenen Bereich um zumindest 360° erstreckt. Die Schenkelfeder kann auch mehrere Windungen 46 aufweisen. Die Schenkel 48 und 50 der Schenkelfeder 44 stehen zu einer gleichen Seite hin von der zumindest einen Windung 46 ab.

Die Federanordnung 42 weist weiterhin einen Zapfen 52 auf, im gezeigten Ausführungsbeispiel weist die Federanordnung 42 zwei Zapfen 52a, 52b auf, die nachfolgend einheitlich auch als Zapfen 52 bezeichnet werden. Die Schenkelfedern 44a und 44b sitzen auf dem Zapfen 52a, und die Schenkelfedern 44c und 44d sitzen auf dem Zapfen 52b. Wie in Fig. 2 in Verbindung mit Fig. 6 zu sehen ist, sind zwei der Schenkelfedern, und zwar die Schenkelfedern 44a, 44b einerseits und die Schenkelfedern 44c und 44d andererseits paarweise beidseits einer Längsachse 80 des Schaftes 12 angeordnet.

Die zumindest eine Windung 46 der Schenkelfeder 44 erstreckt sich um den Zapfen 52 herum. Dabei kann die Windung 46 der Schenkelfeder 44 eng um den Zapfen 52 geschlungen sein, so dass sich die Schenkelfeder 44 im Ruhezustand, d.h. wenn die Schenkel 48, 50 nicht mit einer Kraft beaufschlagt werden, aufgrund eines Reibschlusses nicht relativ zu dem Zapfen 52 drehen kann. Die Schenkelfeder 44 könnte aber auch nur so eng um den Zapfen 52 geschlungen sein, dass sich die Schenkelfeder 44 im Ruhezustand relativ zu dem Zapfen drehen kann.

Der Zapfen 52 ist drehfest an der Handhabe 20 angeordnet. Der Zapfen 52 kann so angeordnet sein, wie im vorliegenden Ausführungsbeispiel gezeigt, dass er mit der Drehachse 25 fluchtet. Der Zapfen 52 kann einen Fortsatz 53 aufweisen, über den der Zapfen 52 an dem unbeweglichen Griffteil 22 an einer Anlenkungsstelle 57 drehfest festgelegt ist, an der das bewegliche Griffteil 24 beweglich gelagert ist. Der Fortsatz 53 kann durch eine Öffnung 54 in einem Abschnitt 56 des beweglichen Griffteils 24 hindurchgeführt sein. Der Fortsatz 53 kann als Welle für das bewegliche Griffteil 24 dienen.

Die Aufnahme 36 für die Festlegung des proximalen Endes des Kraftübertragungselements 32 weist Blöcke 58a und 58b auf, die fest, beispielsweise einstückig, mit der Aufnahme 36 verbunden sind. Die Blöcke 58a und 58b werden nachfolgend einheitlich auch als Block 58 bezeichnet. Der Schenkel 50 der Schenkelfeder 44 ist an dem Block 58 mittels eines Befestigungselements 60 festgelegt. Das Befestigungselement 60 kann ein Unterlegblech 62 und ein oder mehrere Schrauben 64 aufweisen, die in Gewindebohrungen 66 am Block 58 eingeschraubt sind. Der Schenkel 50 ist entsprechend zwischen dem Unterlegblech 62 und dem Block 58 eingeklemmt. Für den Schenkel 48 ist ein Befestigungselement 67 mit einem Unterlegblech 68 und einer oder mehreren Schrauben 70 vorgesehen, um den Schenkel 48 auf der dem Schenkel 50 gegenüberliegenden Seite des Blocks 58 an diesem festzulegen. Auf diese Weise sind die Schenkel 48, 50 mit der Aufnahme 36 über den Block 58 fest verbunden.

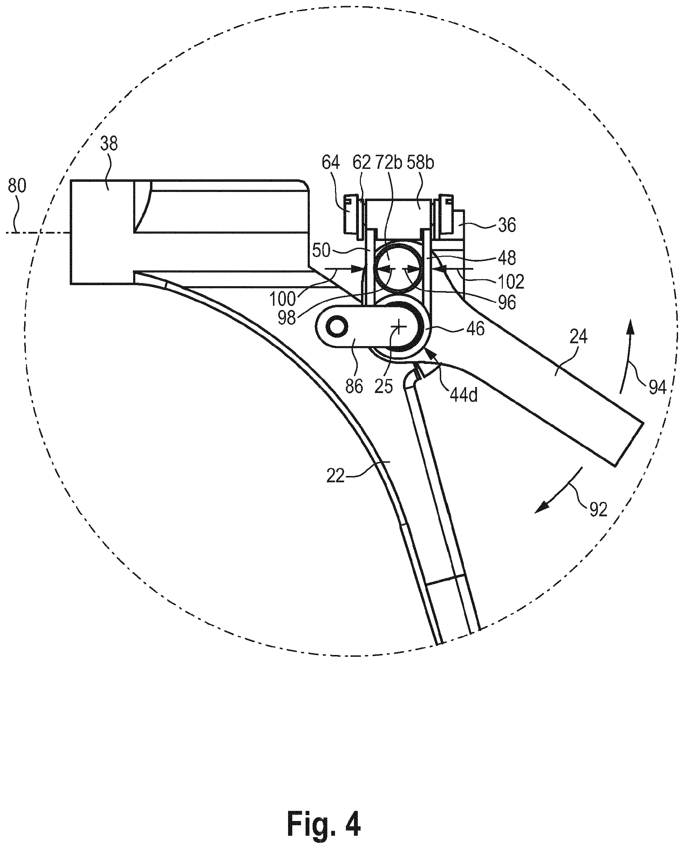

Wie bspw. in Fig. 4 zu sehen ist, sind die Schenkel 48 und 50 der Schenkelfeder 44 in Längsrichtung des Schaftes gesehen axial hintereinander angeordnet und axial voneinander beabstandet. In dem gezeigten Ausführungsbeispiel verlaufen die Schenkel 48 und 50 parallel zueinander.

Der Selbsthemmungsmechanismus 40 weist weiterhin einen Mitnehmer 72 auf, wobei im vorliegenden Ausführungsbeispiel zwei Mitnehmer 72a und 72b, die hiernach einheitlich auch als Mitnehmer 72 bezeichnet werden, vorhanden sind.

Der Mitnehmer 72 ist an dem beweglichen Griffteil 24 exzentrisch zur Drehachse 25 des beweglichen Griffteils 24 angeordnet. Bei einer Verschwenkung des beweglichen Griffteils 24 wird der Mitnehmer 72 mitbewegt. Im vorliegenden Ausführungsbeispiel weist das bewegliche Griffteil 24 im Gabelabschnitt 56 eine Bohrung 74 für die Befestigung des Mitnehmers 72 auf. Der Mitnehmer 72 ist zwischen den Schenkeln 48 und 50 der Schenkelfeder 44 angeordnet, wie in Fig. 4 zu sehen ist.

Die Aufnahme 36 mit dem Block 58 ist an dem beweglichen Griffteil 24 drehbeweglich befestigt und weist hierzu ein Auge 75 auf. Die Aufnahme 36 ist beispielsweise über einen Fortsatz 73 des Mitnehmers 72, der durch eine Bohrung 74 an dem Abschnitt 56 in eine Bohrung 75 an der Aufnahme 36 greift, mit dem beweglichen Griffteil 24 fest verbunden. Bei einer Verschwenkung des beweglichen Griffteils 24 bewegt sich die Aufnahme 36 und der Mitnehmer 72 somit mit dem beweglichen Griffteil 24 mit, und zwar um die Drehachse 25. Der Zapfen 52 hingegen ist bei einer Verschwenkung des beweglichen Griffteils 24 unbeweglich.

Abschlussbleche 86 sind an den seitlich äußeren Enden der Zapfen 52a, 52b befestigt und bilden zusammen mit einer durch das feste Griffteil 22 durch eine Bohrung 88 hindurchgehende Stange 90 eine Verwindungssicherung für die Schenkelfeder 44.

Die Funktionsweise des Selbsthemmungsmechanismus 40 wird nachfolgend beschrieben.

Bei einer Betätigung des beweglichen Griffteils 24 greift je nach Richtung der Betätigung des beweglichen Griffteils 24 an dem Schenkel 48 oder an dem Schenkel 50 eine Kraft an, die die Reibung zwischen der Windung 46 und dem Zapfen 52 verringert, so dass die Schenkelfeder 44 um den Zapfen 52 dann drehbeweglich ist. Dies ist in dem Ausführungsbeispiel dadurch realisiert, dass der Mitnehmer 72 bei einer Bewegung des beweglichen Griffteils 24 in Richtung eines Pfeiles 92 in Fig. 4 am Schenkel 48 mit einer Kraft (Pfeil 96) nach proximal drückt, und bei einer Bewegung des beweglichen Griffteils 24 in Richtung eines Pfeiles 94 den Schenkel 50 nach distal drückt (Pfeil 98). In beiden Fällen bewirkt die Betätigung des Griffteils 24, dass sich die Windung 46 im Durchmesser, wenn auch nur geringfügig, vergrößert, wodurch die Reibung zwischen der Windung 46 und dem Zapfen 52 verringert oder sogar ganz aufgehoben wird, wodurch sich die Schenkelfeder 44 auf dem Zapfen 52 drehen kann. Die Betätigung des beweglichen Griffteils 24 wird gleichzeitig in eine Längsbewegung des Kraftübertragungselements 32 nach distal oder nach proximal umgesetzt, je nach Betätigungsrichtung des beweglichen Griffteils 24. Die Längsbewegung des Kraftübertragungselements 32 bewirkt eine Bewegung des Werkzeuges 14 am distalen Ende des Schaftes 12.

Wirkt dagegen eine Kraft auf das Werkzeug 14, die versucht, das Werkzeug 14 zu bewegen, so wird diese Kraft vom Werkzeug 14 auf das Kraftübertragungselement 32 übertragen, und zwar in dessen Längsrichtung, und überträgt sich über die Aufnahme 36 auf die Federanordnung 42, so dass diese Kraft nun nicht innenseitig wie oben beschrieben, sondem außenseitig auf die Schenkel 48 bzw. 50 wirkt (Pfeile 100, 102), wodurch sich die Windung 46 noch enger um den Zapfen 52 schlingt und der zunehmende Reibschluss verhindert, dass sich die Schenkelfeder 44 auf dem Zapfen 52 drehen kann. Somit ist jegliche Beweglichkeit des Werkzeugs 14 und des Kraftübertragungselements 32 gesperrt.

Der Selbsthemmungsmechanismus 40 wirkt unmittelbar bei Auftreten einer Kraft auf das Werkzeug 14, die versucht das Werkzeug 14 zu bewegen. Die Selbsthemmung tritt unabhängig davon ein, ob eine Kraft auf das Werkzeug 14 wirkt, die versucht, das Werkzeug zu öffnen, wie dies der Fall ist, wenn ein Objekt zwischen den Maulteilen 16 und 18 gefasst ist, oder ob die Kraft versucht, die Maulteile 16, 18 zu schließen. In beiden Fällen wird der Reibschluss zwischen der Windung 46 der Schenkelfeder 44 um den Zapfen 52 nicht überwunden. Diese drehfeste Verbindung kann nur durch Betätigen des beweglichen Griffteils 24 aufgehoben werden, indem der Mitnehmer 72 die Schenkel 48 und 50 auseinanderbewegt. Sowohl die Selbsthemmung als auch die freie Gängigkeit des Werkzeuges 14 (bei Betätigen des beweglichen Griffteils 24) wirken bidirektional.

Der Selbsthemmungsmechanismus 40 wirkt insbesondere stufenlos, d.h. das Wirksamwerden der Selbsthemmung hängt nicht von der Stellung des beweglichen Griffteils 24 entlang des möglichen Bewegungsweges des beweglichen Griffteils 24 ab.

Der Selbsthemmungsmechanismus 40 benötigt keinen Lösemechanismus, sondern löst sich selbsttätig wenn das bewegliche Griffteil 24 in der einen oder anderen Bewegungsrichtung (Pfeile 92, 94 in Fig. 4) betätigt wird.

Ein medizinisches Instrument weist einen langerstreckten Schaft 12, ein bewegliches Werkzeug 14 an einem distalen Ende des Schaftes 12, eine Handhabe 20 mit einem beweglichen Griffteil 24 an einem proximalen Ende des Schaftes 12, und ein in seiner Längsrichtung bewegliches Kraftübertragungselement 32, das mit dem beweglichen Griffteil 24 und mit dem Werkzeug 14 in Wirkverbindung steht, so dass eine Betätigung des beweglichen Griffteils 24 in eine Bewegung des Werkzeugs 14 umgesetzt wird. Das Instrument 1 weist eine an der Handhabe 20 angeordnete Federanordnung 42 auf, die zumindest eine Schenkelfeder 44, die zumindest eine Windung 46 und zwei Schenkel 48, 50 aufweist, und einen Zapfen 52 aufweist, wobei sich die zumindest eine Windung 46 um den Zapfen 52 herum erstreckt. Bei einer Betätigung des beweglichen Griffteils 24 greift an zumindest einem der Schenkel 48, 50 der Schenkelfeder 44 eine Kraft an, die die Reibung zwischen der Windung 46 und dem Zapfen 52 verringert, so dass die Schenkelfeder 44 um den Zapfen 52 drehbeweglich ist und sich das Kraftübertragungselement 32 und das Werkzeug 14 bewegen können. Bei einer vom Werkzeug 14 auf das Kraftübertragungselement 32 in dessen Längsrichtung übertragene Kraft ist die Schenkelfeder 44 um den Zapfen 52 nicht drehbeweglich, so dass sich das Kraftübertragungselement 32 und das Werkzeug 14 nicht bewegen können. Es versteht sich, dass die vorstehend genannten und erläuterten Merkmale nicht nur in der jeweils angegebenen Kombination, sondern auch in anderen Kombinationen oder in Alleinstellung verwendbar sind, ohne den Rahmen der vorliegenden Erfindung zu verlasser, solange dies unter den Schutzumfang der Ansprüche fallen.

## Patentansprüche

1. Medizinisches Instrument, mit einem langerstreckten Schaft (12), einem beweglichen Werkzeug (14) an einem distalen Ende des Schaftes (12), einer Handhabe (20) mit einem beweglichen Griffteil (24) an einem proximalen Ende des Schaftes (12), und mit einem in seiner Längsrichtung beweglichen Kraftübertragungselement (32), das mit dem beweglichen Griffteil (24) und mit dem Werkzeug (14) in Wirkverbindung steht, so dass eine Betätigung des beweglichen Griffteils (24) in eine Bewegung des Werkzeugs (14) umgesetzt wird,
**gekennzeichnet durch**
eine an der Handhabe (20) angeordnete Federanordnung (42), die zumindest eine Schenkelfeder (44), die zumindest eine Windung (46) und zwei Schenkel (48, 50) aufweist, und einen Zapfen (52) aufweist, wobei sich die zumindest eine Windung (46) um den Zapfen (52) herum erstreckt, wobei das bewegliche Griffteil (24) bei seiner Betätigung auf zumindest einen der Schenkel (48, 50) der Schenkelfeder (44) eine Kraft ausübt, die die Reibung zwischen der Windung (46) und dem Zapfen (52) verringert, so dass die Schenkelfeder (44) um den Zapfen (52) drehbeweglich ist und sich das Kraftübertragungselement (32) und das Werkzeug (14) bewegen können, wogegen die Schenkelfeder (44) bei einer vom Werkzeug (14) auf das Kraftübertragungselement (32) in dessen Längsrichtung übertragenen Kraft um den Zapfen (52) nicht drehbeweglich ist, so dass sich das Kraftübertragungselement (32) und das Werkzeug (14) nicht bewegen können.

2. Instrument nach Anspruch 1, wobei die zumindest eine Windung (46) der Schenkelfeder (44) eng um den Zapfen (52) geschlungen ist, so dass die Schenkelfeder (44) im Ruhezustand aufgrund Reibung um den Zapfen (52) nicht drehbeweglich ist.

3. Instrument nach Anspruch 1 oder 2, wobei eine vom Werkzeug (14) auf das Kraftübertragungselement (32) in dessen Längsrichtung übertragene Kraft an zumindest einem der Schenkel (48, 50) angreift, wodurch die Reibung zwischen der zumindest einen Windung (46) und dem Zapfen (52) erhöht wird.

4. Instrument nach einem der Ansprüche 1 bis 3, wobei die Längsbewegung des Kraftübertragungselements (32) und die Bewegung des Werkzeugs (14) sowohl gesperrt ist, wenn die über das Werkzeug (14) auf das Kraftübertragungselement (32) wirkende Kraft nach distal gerichtet ist, als auch, wenn die über das Werkzeug (14) auf das Kraftübertragungselement (32) wirkende Kraft nach proximal gerichtet ist.

5. Instrument nach einem der Ansprüche 1 bis 4, wobei in den beiden Betätigungsrichtungen des beweglichen Griffteils (24) an jeweils einem der Schenkel (48, 50) der Schenkelfeder (44) eine Kraft angreift, die die Reibung zwischen der zumindest einen Windung (46) und dem Zapfen (52) verringert.

6. Instrument nach einem der Ansprüche 1 bis 5, wobei das bewegliche Griffteil (24) um eine Drehachse (25) verschwenkbar ist, und wobei der Zapfen (52) mit der Drehachse (25) fluchtet.

7. Instrument nach einem der Ansprüche 1 bis 6, wobei das bewegliche Griffteil (24) einen Mitnehmer (72) aufweist, der bei Betätigung des beweglichen Griffteils (24) auf zumindest einen der Schenkel (48, 50) der Schenkelfeder (44) die Kraft ausübt, um die Reibung zwischen der Windung (46) und dem Zapfen (52) zu verringern.

8. Instrument nach Anspruch 7, wobei die Schenkel (48, 50) der Schenkelfeder (44) von der zumindest einen Windung (46) zur gleichen Seite abstehen, und der Mitnehmer (72) zwischen den Schenkeln (48, 50) der Schenkelfeder (44) angeordnet ist.

9. Instrument nach einem der Ansprüche 1 bis 8, wobei das bewegliche Griffteil (24) eine Aufnahme (36) zur Verbindung eines proximalen Endes des Kraftübertragungselements (32) mit dem beweglichen Griffteil (24) aufweist, und wobei zumindest einer der Schenkel (48, 50) der Schenkelfeder (44) mit der Aufnahme (36) fest verbunden ist.

10. Instrument nach einem der Ansprüche 1 bis 9, wobei die Federanordnung (42) zumindest zwei Schenkelfedern (44b, 44c) aufweist, die beidseits einer Längsmittelachse (80) des Schaftes (12) angeordnet sind.

11. Instrument nach Anspruch 10, wobei die Federanordnung (42) zumindest vier Schenkelfedern (44a, 44b, 44c, 44d) aufweist, die paarweise beidseits der Längsmittelachse (80) des Schaftes (12) angeordnet sind.

12. Instrument nach einem der Ansprüche 1 bis 11, wobei die zumindest eine Schenkelfeder (44) mehrere Windungen aufweist.

13. Verfahren zum Immobilisieren und Freigeben der Beweglichkeit eines beweglichen Werkzeuges (14) eines medizinischen Instruments (10), das einen langerstreckten Schaft (12), das bewegliche Werkzeug (14) an einem distalen Ende des Schaftes (12), eine Handhabe (20) mit einem beweglichen Griffteil (24) an einem proximalen Ende des Schaftes (12), und ein in seiner Längsrichtung bewegliches Kraftübertragungselement (32) aufweist, das mit dem beweglichen Griffteil (24) und mit dem Werkzeug (14) in Wirkverbindung steht, wobei die Beweglichkeit des Werkzeuges (14) und des beweglichen Griffteils (24) unmittelbar unterbunden wird, wenn das bewegliche Griffteil (24) nicht betätigt wird, und wobei die Beweglichkeit des Werkzeuges (14) unmittelbar durch Betätigen des beweglichen Griffteils (24) freigegeben wird.

## Claims

1. A medical instrument comprising an elongated shaft (12), a movable tool (14) at a distal end of the shaft (12), a handle (20) with a movable grip part (24) at a proximal end of the shaft (12), and with a force transmission element (32), which is movable in its longitudinal direction and which is operatively connected to the movable grip part (24) and to the tool (14), such that actuation of the movable grip part (24) is converted into a movement of the tool (14),
**characterised by**
a spring arrangement (42), which is arranged on the handle (20) and which has at least one leg spring (44) having at least one winding (46) and two legs (48, 50), and a pin (52), wherein the at least one winding (46) extends around the pin (52), wherein the movable grip part (24), when actuated, exerts a force on at least one of the legs (48, 50) of the leg spring (44) which reduces the friction between the winding (46) and the pin (52) such that the leg spring (44) is rotatably movable about the pin (52) and the force transmission element (32) and the tool (14) can move, whereas the leg spring (44) is not rotatably movable about the pin (52) when a force is transmitted from the tool (14) to the force transmission element (32) in the longitudinal direction thereof such that the force transmission element (32) and the tool (14) cannot move.

2. The instrument according to claim 1, wherein the at least one winding (46) of the leg spring (44) is wound tightly around the pin (52) such that the leg spring (44) is not rotatably movable about the pin (52) in the rest state due to friction.

3. The instrument according to claim 1 or 2, wherein a force transmitted from the tool (14) to the force transmission element (32) in the longitudinal direction thereof acts on at least one of the legs (48, 50), thereby increasing the friction between the at least one winding (46) and the pin (52).

4. The instrument according to one of claims 1 to 3, wherein the longitudinal movement of the force transmission element (32) and the movement of the tool (14) is blocked both when the force acting on the force transmission element (32) via the tool (14) is directed distally and when the force acting on the force transmission element (32) via the tool (14) is directed proximally.

5. The instrument according to one of claims 1 to 4, wherein in the two actuating directions of the movable grip part (24) a force acts on each one of the legs (48, 50) of the leg spring (44), which reduces the friction between the at least one winding (46) and the pin (52).

6. The instrument according to one of claims 1 to 5, wherein the movable grip part (24) is pivotable about an axis of rotation (25), and wherein the pin (52) is aligned with the axis of rotation (25).

7. The instrument according to one of claims 1 to 6, wherein the movable grip part (24) comprises a driver (72) which, when the movable grip part (24) is actuated, exerts the force on at least one of the legs (48, 50) of the leg spring (44) to reduce the friction between the winding (46) and the pin (52).

8. The instrument according to claim 7, wherein the legs (48, 50) of the leg spring (44) project from the at least one winding (46) to the same side, and the driver (72) is arranged between the legs (48, 50) of the leg spring (44).

9. The instrument according to one of claims 1 to 8, wherein the movable grip part (24) comprises a receptacle (36) for connecting a proximal end of the force transmission element (32) to the movable grip part (24), and wherein at least one of the legs (48, 50) of the leg spring (44) is fixedly connected to the receptacle (36).

10. The instrument according to one of claims 1 to 9, wherein the spring arrangement (42) comprises at least two leg springs (44b, 44c) which are arranged on both sides of a longitudinal centre axis (80) of the shaft (12).

11. The instrument according to claim 10, wherein the spring arrangement (42) comprises at least four leg springs (44a, 44b, 44c, 44d) which are arranged in pairs on both sides of the longitudinal centre axis (80) of the shaft (12).

12. The instrument according to one of claims 1 to 11, wherein the at least one leg spring (44) comprises a plurality of windings.

13. A method for immobilising and releasing the movability of a movable tool (14) of a medical instrument (10) which has an elongated shaft (12), the movable tool (14) at a distal end of the shaft (12), a handle (20) with a movable grip part (24) at a proximal end of the shaft (12), and a force transmission element (32), which is movable in its longitudinal direction and which is operatively connected to the movable grip part (24) and to the tool (14), wherein the movability of the tool (14) and of the movable grip part (24) is immediately prevented when the movable grip part (24) is not actuated, and wherein the movability of the tool (14) is immediately released by actuating the movable grip part (24).

## Revendications

1. Instrument médical, comportant une tige allongée (12), un outil mobile (14) sur une extrémité distale de la tige (12), une poignée (20) comportant une partie de préhension mobile (24) sur une extrémité proximale de la tige (12), et comportant un élément de transmission de force (32) mobile dans sa direction longitudinale, qui est en liaison active avec la partie de préhension mobile (24) et avec l'outil (14), de sorte qu'un actionnement de la partie de préhension mobile (24) est converti en un mouvement de l'outil (14),
**caractérisé par**
un agencement de ressort (42) agencé sur la poignée (20), qui présente au moins un ressort à branches (44), qui présente au moins une spire (46) et deux branches (48, 50), et un tourillon (52), dans lequel l'au moins une spire (46) s'étendant autour du tourillon (52), dans lequel la partie de préhension mobile (24) exerce, lors de son actionnement, sur au moins l'une des branches (48, 50) du ressort à branches (44) une force qui réduit le frottement entre la spire (46) et le tourillon (52), de sorte que le ressort à branches (44) est mobile en rotation autour du tourillon (52) et l'élément de transmission de force (32) et l'outil (14) peuvent entrer en mouvement, tandis que le ressort à branches (44) n'est pas mobile en rotation autour du tourillon (52) lors d'une force transmise par l'outil (14) à l'élément de transmission de force (32) dans sa direction longitudinale, de sorte que l'élément de transmission de force (32) et l'outil (14) ne peuvent pas entrer en mouvement.

2. Instrument selon la revendication 1, dans lequel l'au moins une spire (46) du ressort à branches (44) est étroitement enroulée autour du tourillon (52), de sorte que le ressort à branches (44) n'est pas mobile en rotation autour du tourillon (52) en raison du frottement à l'état de repos.

3. Instrument selon la revendication 1 ou 2, dans lequel une force transmise par l'outil (14) à l'élément de transmission de force (32) dans sa direction longitudinale s'applique à au moins l'une des branches (48, 50), moyennant quoi le frottement entre l'au moins une spire (46) et le tourillon (52) est accrue.

4. Instrument selon l'une quelconque des revendications 1 à 3, dans lequel le mouvement longitudinal de l'élément de transmission de force (32) et le mouvement de l'outil (14) sont bloqués à la fois lorsque la force agissant sur l'élément de transmission de force (32) par l'intermédiaire de l'outil (14) est dirigée vers le côté distal et lorsque la force agissant sur l'élément de transmission de force (32) par l'intermédiaire de l'outil (14) est dirigée vers le côté proximal.

5. Instrument selon l'une des revendications 1 à 4, dans lequel, dans les deux directions d'actionnement de la partie de préhension mobile (24), une force est appliquée respectivement sur l'une des branches (48, 50) du ressort à branches (44), ce qui réduit le frottement entre l'au moins une spire (46) et le tourillon (52).

6. Instrument selon l'une des revendications 1 à 5, dans lequel la partie de préhension mobile (24) peut pivoter autour d'un axe de rotation (25), et dans lequel le tourillon (52) est aligné avec l'axe de rotation (25).

7. Instrument selon l'une des revendications 1 à 6, dans lequel la partie de préhension mobile (24) présente un entraîneur (72) qui, lors de l'actionnement de la partie de préhension mobile (24), exerce sur au moins l'une des branches (48, 50) du ressort à branches (44) la force permettant de réduire le frottement entre la spire (46) et le tourillon (52).

8. Instrument selon la revendication 7, dans lequel les branches (48, 50) du ressort à branches (44) dépassent de l'au moins une spire (46) du même côté, et l'entraîneur (72) est agencé entre les branches (48, 50) du ressort à branches (44).

9. Instrument selon l'une des revendications 1 à 8, dans lequel la partie de préhension mobile (24) présente un logement (36) pour relier une extrémité proximale de l'élément de transmission de force (32) à la partie de préhension mobile (24), et dans lequel au moins l'une des branches (48, 50) du ressort à branches (44) est solidaire du logement (36).

10. Instrument selon l'une des revendications 1 à 9, dans lequel l'agencement de ressort (42) présente au moins deux ressorts à branches (44b, 44c), qui sont agencés de part et d'autre d'un axe longitudinal médian (80) de la tige (12).

11. Instrument selon la revendication 10, dans lequel l'agencement de ressort (42) présente au moins quatre ressorts à branches (44a, 44b, 44c, 44d), qui sont agencés par paires de part et d'autre de l'axe longitudinal médian (80) de la tige (12).

12. Instrument selon l'une des revendications 1 à 11, dans lequel l'au moins un ressort à branches (44) présente plusieurs spires.

13. Procédé d'immobilisation et de libération de la mobilité d'un outil mobile (14) d'un instrument médical (10), qui présente une tige allongée (12), l'outil mobile (14) sur une extrémité distale de la tige (12), une poignée (20) comportant une partie de préhension mobile (24) sur une extrémité proximale de la tige (12), et un élément de transmission de force (32) mobile dans sa direction longitudinale, qui est en liaison active avec la partie de préhension mobile (24) et avec l'outil (14), dans lequel la mobilité de l'outil (14) et de la partie de préhension mobile (24) sont immédiatement empêchée lorsque la partie de préhension mobile (24) n'est pas actionnée, et dans lequel la mobilité de l'outil (14) est immédiatement libérée par actionnement de la partie de préhension mobile (24).
